# EUROPÄISCHE PATENTSCHRIFT

(11) **EP 3 535 004 B1**
(45) Veröffentlichungstag und Bekanntmachung des Hinweises auf die Patenterteilung: **03.03.2021**
(21) Anmeldenummer: 17797263.5
(22) Anmeldetag: 25.10.2017
(51) Int. Cl.: A61M 16/04

(54) **TRACHEALKANÜLE MIT EINER ABDICHTEINRICHTUNG**
TRACHEAL CANNULA WITH A SEALING DEVICE
CANULE TRACHÉALE DOTÉE D'UN DISPOSITIF D'ÉTANCHÉITÉ

(30) Priorität: 02.11.2016 DE 102016120819
(43) Veröffentlichungstag der Anmeldung: 11.09.2019
(73) Patentinhaber: Tracoe Medical GmbH, 55268 Nieder-Olm (DE)
(72) Erfinder: SCHNELL, Ralf, 63500 Seligenstadt (DE)
(74) Vertreter: WSL Patentanwälte Partnerschaft mbB
(86) Internationale Anmeldenummer: PCT/EP2017/077260
(87) Internationale Veröffentlichungsnummer: WO 2018/082981

(56) Entgegenhaltungen:
- WO-A1-2008/009947
- US-A- 3 995 643
- US-A1- 2006 278 235
- US-A1- 2010 089 404
- US-A1- 2010 288 289
- US-A1- 2011 073 115
- US-A1- 2016 101 253

## Beschreibung

Die vorliegende Erfindung betrifft eine Trachealkanüle, insbesondere eine Tracheostomiekanüle mit einem Tubus, wobei der Tubus ein proximales Ende mit einer proximalen Öffnung, ein distales Ende mit einer distalen Öffnung und eine an seinem äußeren Umfang nahe des distalen Ende angeordnete Abdichteinrichtung aufweist.

Darüber hinaus betrifft die vorliegende Erfindung auch eine Vorrichtung zur Atemluftzuführung, die aus zwei ineinander gesteckten Trachealkanülen besteht.

Trachealkanülen werden in die Trachea eines Patienten eingeführt, um den Patienten mit Luft bzw. mit Atemgas zu versorgen. Das proximale Ende der Trachealkanüle ist außerhalb des Patienten mit einer Beatmungsvorrichtung verbindbar, während das distale Ende der Trachealkanüle in die Trachea einführbar ist. Der die zwei Enden der Trachealkanüle verbindende Tubus, dient der Hindurchleitung von Atemgas.

Für eine zuverlässige Versorgung mit Atemgas ist es wichtig, dass das durch den Tubus in die Trachea eingeleitete Atemgas vor allem beim Zuführen nicht zurück und an dem Tubus vorbei durch die Trachea und Mund und Rachen des Patienten strömt, bevor es in die Lunge gelangt. Darüber hinaus gilt es zu vermeiden, dass Sekret aus dem subglottischen Raum an dem in die Trachea eingeführten Tubus vorbei in die Bronchien des Patienten gelangt. Das Sekret enthält zumeist Bakterien, die bei einer Aspiration zu Pneumonien führen können.

Herkömmliche Trachealkanülen weisen daher auf der Außenseite ihrer Tuben eine Abdichteinrichtung in Form eines ballonartigen Cuffs auf, der die Trachealkanüle gegenüber der Innenwand der Trachea abdichtet. Die Trachealkanüle wird in einem Zustand, in dem der Cuff leer und eng an dem Tubus anliegt, in die Trachea eines zu behandelnden Patienten eingeführt. Anschließend wird der aus flexiblem Weichfolienmaterial bestehende Cuff von innen mit einem Fluid beaufschlagt, das den ballonartigen Cuff aufweitet und gegen die Innenwand der Trachea drückt. Um die Abdichtfunktion durch die Manschette zu gewährleisten ohne jedoch allzu viel Druck auf die Innenwand der Trachea auszuüben, ist die Einstellung eines angemessenen Fülldruckes entscheidend. Bei einem zu niedrigen Druck, liegt die Manschette nicht ausreichend fest an der Innenwand der Trachea an. Die angestrebte Abdichtfunktion wird nicht erfüllt. Ist der Druck der Manschette zu hoch eingestellt, drückt die Manschette zu fest auf die Trachealschleimhaut und verringert oder unterbricht die Blutversorgung der Trachealschleimhaut. In Extremfällen können auf diese Weise Drucknekrosen entstehen.

Um eine dauerhafte Abdichtung durch den Cuff zu gewährleisten, muss der eingestellte Fülldruck der Manschette wiederholt überprüft und unter Umständen nachreguliert werden. Abgesehen von dem hierfür notwendigen Personalaufwand und den damit verbundenen Kosten ist die wiederholte Überprüfung des Manschettendruckes für den zu behandelnden Patienten unangenehm.

Aus der US 2006/0278235 A1 ist ein Tracheostomietubus bekannt, welcher gemäß Figur 9 dieses Dokumentes als proximale Begrenzung eines Cuffs eine trichterförmig eingesenkte Struktur mit einem Dichtungsrand aufweist, wobei dieser trichterförmige Aufbau zur Aufnahme von Sekret dient, das sich oberhalb des Cuffs sammelt und wobei die zentrale Kanüle Absaugeinrichtungen aufweist, die am Grund der trichterförmigen Struktur münden.

Aus den Dokumenten US 2011/0073115 A1, WO 2008/009974 A1, US 2016/0101253 A1, US 2010/0089404 A1, US 2010/0288289 A1 und US 3,991,643 A sind ebenfalls bereits Tracheostomiekanülen bekannt, die in irgendeiner Weise trichterförmig geformte Strukturen mit Dichtungsrand oberhalb eines Dichtungscuffs aufweisen.

Bei all diesen Strukturen ist jedoch nicht sichergestellt, dass der Dichtungsrand einer solchen trichterförmigen Struktur auch unter Beatmungsdruck, bei welchem beispielsweise auch der Cuff kurzzeitig nicht mehr dicht abschließt, die Funktion des Dichtungsrandes gewährleistet ist. Demgegenüber ist es eine Aufgabe der vorliegenden Erfindung eine Trachealkanüle mit einer Abdichteinrichtung bereitzustellen, die keinen oder nur einen geringen Wartungsaufwand erfordert. und welche die Abdichtfunktion der Ringmanschette auch unter Beatmungsdruck verbessert. Diese Aufgabe wird durch eine Trachealkanüle gemäß Anspruch 1 gelöst. Diese Abdichteinrichtung weist eine ringförmig umlaufende und trichterförmig nach außen und zum proximalen Ende gewölbte Ringmanschette auf, deren distales Ende den Tubus dicht umfasst und deren freies, proximales Ende als umlaufender, elastisch nachgiebiger Dichtrand ausgebildet ist, wobei die Ringmanschette distal unterhalb ihres proximalen Endes zusätzlich zu dem proximal darüber befindlichen Dichtrand eine umlaufende, in Richtung des distalen Endes der Ringmanschette eingerollte Dichtlippe aufweist. Hierdurch wird die Abdichtfunktion der trichterförmigen Manschette gegenüber der Innenwand einer Trachea auch unter Beatmungsdruck weiter verbessert.

Die eingerollte Dichtlippe ist in dem Bereich des gekrümmten Übergangsabschnittes angeordnet und wird unten noch genauer erläutert.

Gemäß dem der Erfindung zu Grunde liegenden Gedanken ist die Trachealkanüle dazu geeignet, mit dem distalen Ende voraus in die Trachea eines Patienten eingeführt zu werden. Das proximale Ende der Trachealkanüle ist für den Anschluss an eine Beatmungsvorrichtung außerhalb des Körpers eines Patienten vorgesehen.

Dabei bezeichnet das "distale Ende" der jeweiligen Kanüle im Sinne der vorliegenden Erfindung das in die Trachea eingeführte bzw. in die Trachea einführbare Ende, das von einem behandelnden Arzt oder medizinischem Personal weiter entfernt liegt als das proximale Ende. Die Begriffe "distal" (fern gelegen) und "proximal" (nahe gelegen) werden also aus der Sicht einer behandelnden Person definiert. Entsprechend ist das "proximale Ende" für die Anordnung außerhalb des Körpers eines Patienten vorgesehen.

Bedingt durch die nach außen und zum proximalen Ende gewölbte Trichterform ist der Außendurchmesser des distalen Endes der Ringmanschette kleiner als der Außendurchmesser des proximalen Endes der Ringmanschette. Das distale Ende geht in den Tubus über und schließt dicht mit diesem ab. Der kleinere Außendurchmesser des distalen Endes erleichtert die Einführung der Trachealkanüle in die Trachea eines Patienten.

Das freie proximale Ende der Ringmanschette ist dafür vorgesehen, an der Innenwand der Trachea eines Patienten anzuliegen. Beim Einführen der Trachealkanüle in eine Trachea kann der umlaufende, elastisch nachgiebige Dichtrand radial nach innen ausweichen und sich an den Tubus anlegen und sich schließlich in der Trachea wieder aufweiten und an der Innenwand der Trachea entlanggleiten. Der umlaufende, nachgiebige Dichtrand verhindert in einem Zustand, in dem er an der Innenwand der Trachea eines Patienten anliegt, dass Sekret, beispielsweise aus dem subglottischen Raum, an der Trachealkanüle vorbei in die Bronchien gelangt. Es versteht sich, dass der Dichtrand hierfür nicht gasdicht an der Trachea anliegen muss. Vielmehr kann ein geringer Luftstrom an dem Dichtrand vorbeiströmen.

Sekret, welches an der Glottis vorbei in die distale Richtung läuft, gelangt aufgrund der mit einem Dichtrand versehenen Ringmanschette nicht in die Bronchien, sondern in den Zwischenraum zwischen der trichterförmigen Ringmanschette und dem Tubus. Da das distale Ende der Ringmanschette den Tubus dicht umfasst, wird das Sekret in dem Zwischenraum zwischen der trichterförmigen Ringmanschette und dem Tubus gesammelt und gelangt somit nicht in die Bronchien.

In einer Ausführungsform hat der Zwischenraum zwischen der trichterförmigen Ringmanschette und dem Tubus ein Volumen zwischen 2 ml und 10 ml.

In einer Ausführungsform ist die trichterförmige Ringmanschette elastisch leicht verformbar. Eine elastisch verformbare Ringmanschette erleichtert das Entfernen der Trachealkanüle aus der Trachea eines Patienten. Beim Zurückziehen der Trachealkanüle, d.h. beim Herausziehen der Trachealkanüle aus einer Trachea in die proximale Richtung, kann zumindest ein Teil der elastisch verformbaren Ringmanschette, insbesondere der an dem proximalen Ende angeordnete Dichtrand, in die distale Richtung umklappen, umbiegen bzw. einrollen. In einer Ausführungsform kann die komplette Ringmanschette bei Entnahme der Trachealkanüle umklappen. Durch ein Umklappen, Umbiegen oder Einrollen verkleinert sich der Außendurchmesser des proximalen Endes der Ringmanschette, sodass insgesamt die Trachealkanüle einfacher aus der Trachea eines Patienten entfernt werden kann. Bedingt durch die elastische Verformbarkeit ist die Ringmanschette durch Engstellen hindurchführbar und lässt sich nach einer vorübergehenden Verformung in ihre Ausgangsform zurückverformen.

Die leichte Verformbarkeit soll gewährleisten, dass die Trachea durch den anliegenden Dichtrand nicht übermäßig beansprucht oder gar verletzt wird. Der Dichtrand kann in einer Ausführungsform die Form einer Dichtlippe haben, wird aber hier zur Unterscheidung gegenüber einer weiteren, noch zu beschreibenden Dichtlippe generell als "Dichtrand" bezeichnet.

Um die Abdichtfunktion der trichterförmigen Ringmanschette zu verbessern, ist der elastische Dichtrand in einer Ausführungsform radial nach außen und vorzugsweise auch in Umfangsrichtung in der Trachea vorgespannt. Durch die elastische radiale Vorspannung wird ein dichtes Anlegen des Dichtrandes an der Wand der Trachea sichergestellt. Die Vorspannung in Umfangsrichtung stellt sicher, dass im Falle einer Durchmesseränderung des Dichtrandes, insbesondere bei einer Reduzierung des Durchmessers aufgrund des Eingriffs mit der Trachea, keine Falten am Dichtrand entstehen, die ansonsten entstehen könnten.

In einer weiteren Ausführungsform weist der elastische Dichtrand zumindest einen Versteifungsabschnitt auf, wobei sich zumindest ein Versteifungsabschnitt entlang des radial innenliegenden Randes des Dichtrandes und/oder ein Versteifungsabschnitt parallel zu und im Abstand von dem radial außenliegenden Rand des Dichtrandes erstreckt. Der zumindest eine Versteifungsabschnitt erhöht die Stabilität des ansonsten relativ weichelastischen Dichtrandes und verhindert, dass der Dichtrand in Folge einer Bewegung der Trachea oder einer Ansammlung von Sekret auf dem Dichtrand umklappt oder einknickt. In einer Ausführungsform weist der elastische Dichtrand zumindest einen Versteifungsabschnitt in Form einer Vielzahl von sich radial erstreckender Versteifungsrippen auf. Vorzugsweise sind die sich radial erstreckenden Versteifungsabschnitte äquidistant entlang des Umfangs des Tubus verteilt angeordnet und enden im Abstand zu dem radial äußeren Rand des Dichtrandes.

In einer Ausführungsform ist der elastische Dichtrand ein Dichtwulst aus einem weichelastischen Material, vorzugsweise in Torusform. Ein weichelastisches Material im Sinne der vorliegenden Erfindung ist vorzugsweise ein Elastomer oder ein Polymer mit einer Shore-A-Härte im Bereich von 3 bis 60. Vorzugsweise ist das weichelastische Material ein thermoplastisches Elastomer, wie beispielsweise Styrol-Ethylen-Butylen-Styrol (SEBS), ein geschlossenporiger thermoplastisches Elastomer oder ein Silikon. In einer Ausführungsform hat der Dichtrand eine Shore-A-Härte in einem Bereich von 3 bis 60, vorzugsweise in einem Bereich von 10 bis 40. In einer Ausführungsform bestehen die trichterförmige Ringmanschette und der Dichtrand aus einem einheitlichen Material. Der Durchmesser, die Materialstärke und die Shore-Härte des Dichtrandes sind derart aufeinander abgestimmt, dass der Dichtrand mit einer Vorspannung an der Trachea anliegt. Vorzugsweise entspricht die Vorspannung einem Druck von 5 mbar bis 30 mbar, mit dem der Dichtrand auf die Trachea drückt.

In einer Ausführungsform definiert der radial äußere Rand des Dichtrandes in der Draufsicht entlang der Tubusachse eine kreisförmige Kontur, eine ovale Kontur oder eine D-förmige Kontur mit abgerundeten Ecken, die an die Kontur eines einen typischen Tracheaquerschnitts angenähert ist. Vorzugsweise weist das distale Ende der Trachealkanüle eine Markierung auf, die die Orientierung der trichterförmigen Ringmanschette relativ zu der Längsachse der Trachealkanüle anzeigt. Mit Hilfe der Markierung kann die Querschnittsform der trichterförmigen Ringmanschette in der Trachea ausgerichtet werden.

Vorzugsweise ist die trichterförmige Ringmanschette mit dem Tubus verschweißt, angespritzt, verklebt oder in einem Stück aus dem Material des Tubus ausgeformt. Durch den Übergang der trichterförmigen Manschette in das Material des Tubus wird eine feste und dichte Verbindung zwischen Tubus und Ringmanschette erreicht. Durch die feste und dichte Verbindung wird ein distal geschlossener Zwischenraum zwischen der trichterförmigen Ringmanschette und dem Tubus gebildet, in dem sich Sekret ansammeln und z. B. durch eine Absaugeinrichtung entfernt werden kann. Vorzugsweise sind die trichterförmige Ringmanschette und/oder der Tubus aus thermoplastischem Elastomer, wie beispielsweise SEBS, einem geschlossenporigen thermoplastischen Elastomer oder Silikon hergestellt.

In einer weiteren Ausführungsform weist die Ringmanschette zwischen ihrem proximalen Ende und ihrem distalen Ende einen in axialer Längsrichtung gekrümmten Übergangsabschnitt auf. Der gekrümmte Übergangsabschnitt erhöht die Stabilität der Ringmanschette und lässt herabfließendes Sekret radial nach innen und unten in den Zwischenraum fließen.

Die Dichtlippe ist radial nach innen und in die distale Richtung eingerollt, so dass sie durch den von dem distalen Ende der Kanüle her wirkenden Beatmungsdruck radial nach außen und in proximale Richtung entrollt wird, bis sie an der Wand der Trachea anliegt. Das Atemgas drückt also die eingerollte Dichtlippe auseinander. Die eingerollte Dichtlippe erweitert sich dadurch in radialer Richtung und der radial äußere Abschnitt der Dichtlippe wird durch den Atemdruck an die Innenwand der Trachea gedrückt, sodass die Dichtlippe dem Beatmungsdruck von der trichterförmigen Ringmanschette abhält. Dadurch wird verhindert, dass der Beatmungsdruck den Dichtrand der Ringmanschette radial nach innen drückt, wodurch Atemgas über Mund und Rachen des Patienten verloren gehen würde und Sekret an dem Dichtrand in Richtung Lunge vorbeitreten könnte. Sinkt der Atemdruck, rollt sich die Dichtlippe wieder ein. Im Wechsel mit dem Einatmen und Ausatmen sinkt und steigt der Atemdruck, sodass sich die eingerollte Dichtlippe im Wechsel ausrollen und wieder einrollen kann. Auf diese Weise drückt die Dichtlippe im Takt mit dem Beatmungsdruck auf die Innenwand der Trachea. Drucknekrosen an der Innenwand der Trachea werden durch den nur zeitweisen Kontakt vermieden.

In einer Ausführungsform ist die Dichtlippe an der Außenseite der Ringmanschette angeformt. Vorzugsweise ist die Dichtlippe mit der Ringmanschette verschweißt, verklebt, angespritzt oder in einem Stück aus dem Material der Ringmanschette geformt. Weist die Ringmanschette einen in Längsrichtung gekrümmten Übergangsabschnitt auf, so ist die eingerollte Dichtlippe vorzugsweise in diesem Bereich angebracht.

In einer weiteren Ausführungsform sind das Material und die Wandstärke der eingerollten Dichtlippe derart gewählt, dass sich die Dichtlippe bei einem Auftreffen eines sich von distal nach proximal bewegenden Fluidstroms mit einem Druck ≥ 3 mbar, vorzugsweise einem Druck ≥ 15 mbar, aufweitet und sich derart an die Innenwand einer Trachea anlegt. Auf diese Weise wird eine deutliche Abdichtung der Trachea erreicht, wobei das Verhältnis der Beatmungsluft, die bei einem expiratorischen Druck von 20 mbar in den subglottischen Bereich strömt, zu der einströmenden Luft kleiner gleich 60% ist. Die Materialstärke der Dichtlippe liegt bei einer Ausführungsform in einem Bereich von 0,1 mm bis 2 mm, vorzugsweise in einem Bereich von 0,2 mm bis 0,6 mm.

In einer Ausführungsform erstreckt sich die Dichtlippe in ihrem eingerollten Zustand in einer Querschnittsansicht näherungsweise spiralförmig über einen Winkel von weniger als 360°, vorzugsweise weniger als 300° oder weniger als 270°, aber mehr als 180°, vorzugsweise mehr als 200° und besonders bevorzugt mehr als 225°.

In einer Ausführungsform ist die Weichheit der eingerollten Dichtlippe und/oder der Dichtrand derart ausgestaltet, dass die eingerollte Dichtlippe und/oder der Dichtrand bei einem expiratorischen Druck ≥ 30 mbar in proximale Richtung umklappt und in dieser Position verbleibt. Befindet sich die eingerollte Dichtlippe und/oder der Dichtrand in einem umgeklappten Zustand, ist der Ausatemwiderstand reduziert und der Patient kann weitestgehend frei ausatmen. Insbesondere bei einem Ausatemstoß, beispielsweise durch einen plötzlichen Hustenanfall, wird die eingerollte Dichtlippe und/oder der Dichtrand nach proximal umgeklappt, sodass der Ausatemwiderstand zumindest kurzzeitig verringert ist. Auf diese Weise wird ein Abhusten erleichtert und gleichzeitig verhindert, dass die Trachealkanüle bedingt durch den plötzlichen Druckanstieg übermäßig in der Trachea bewegt wird.

In einer Ausführungsform weist die Ringmanschette 1 mm bis 5 mm proximal zu der eingerollten Dichtlippe eine Mehrzahl von Durchbrechungen in der Ringmanschettenwand auf, wobei die Durchbrechungen vorzugsweise einen Durchmesser in einem Bereich von 0,2 mm bis 2 mm, vorzugsweise in einem Bereich von 0,5 mm bis 1,5 mm, aufweisen. Die Dichtlippe bildet ähnlich wie die Ringmanschette eine trichterartige Struktur und kann somit auch das durch die Ringmanschette nach innen geleitete und durch die Durchbrechungen abfließende Sekret auffangen. Sekret welches sich zwischen proximal der eingerollten Dichtlippe und distal des umlaufenden Dichtrandes sammelt, kann durch die Durchbrechungen in den Zwischenraum zwischen der Ringmanschette und dem Tubus gelangen und durch die nachfolgend beschriebene Spül- und/oder Absaugöffnung entfernt werden. Für ein Abtransport von Sekret ist es ausreichend, wenn dieses in den Zwischenraum zwischen der eingerollten Dichtlippe und dem umlaufenden Dichtrand gelangt, von wo es über die Spül- und/oder Absaugöffnung entfernt werden kann. Auf diese Weise wird einem Patienten auch das Abhusten von Sekret aus der Lunge vereinfacht.

Um in dem Zwischenraum zwischen der trichterförmigen Ringmanschette und dem Tubus befindliches Sekret einfach entfernen zu können, ohne dass hierfür auch die Trachealkanüle entfernt werden müsste, weist der Tubus in einer Ausführungsform auf seiner Außenseite eine Spül- und/oder Absaugöffnung in einem Bereich zwischen dem proximalen Ende der Ringmanschette und dem distalen Ende der Ringmanschette auf. Durch die Spül- und/oder Absaugöffnung kann in dem Zwischenraum zwischen der Ringmanschette und dem Tubus befindliches Sekret abgesaugt oder die Ringmanschette gespült werden. Die bei einer Spülung verwendete Spülflüssigkeit wird von der trichterförmigen Ringmanschette aufgefangen und kann durch die Spül- und/oder Absaugöffnung abgesaugt werden, sodass die Spülflüssigkeit nicht in die Bronchien gelangen kann.

Vorzugsweise ist die Spül- und/oder Absaugöffnung proximal von dem distalen Ende der Ringmanschette angeordnet und zwar in der Nähe des tiefsten Punktes des Trichterförmigen Zwischenraumes. An dieser Position kann nahezu das gesamte im Zwischenraum zwischen der trichterförmigen Ringmanschette und dem Tubus befindliches Sekret abgesaugt werden. Durch die zwischen der Trachea und der Spül- und/oder Absaugöffnung angeordnete trichterförmige Manschette besteht auch bei einem starken Absaugvakuum kein Risiko, dass die Trachealschleimhaut durch das angelegte Vakuum geschädigt wird. Die Spül- und/oder Absaugöffnung ist über einen Spülkanal mit einer außerhalb des Patienten angeordneten Spül- und/oder Absaugvorrichtung verbindbar. Vorzugsweise ist der Spülkanal mit dem Tubus verklebt oder in die Wand des Tubus integriert. In einer Ausführungsform geht der Spül- bzw. Absaugkanal an dem proximalen Ende der Trachealkanüle in einen Schlauchfortsatz zum Verbinden mit einer Spül- und/oder Absaugvorrichtung über.

In einer Ausführungsform weist die Trachealkanüle ein axial luftdurchlässiges Gelenk auf, wobei das Gelenk das proximale Ende und das distale Ende des Tubus (Kanülenrohrs) miteinander verbindet und mindestens eine begrenzte Beweglichkeit und insbesondere Verschwenkbarkeit der Achse des distalen Endes bezüglich der Achse des proximalen Endes ermöglicht. Das proximale Ende des Tubus wird auch als proximaler Rohrabschnitt und das distale Ende als distaler Rohrabschnitt bezeichnet.

Durch das Gelenk sind das proximale Ende und das distale Ende des Tubus relativ zueinander bewegbar, d. h. die Achsen der beiden Enden des Tubus sind verschwenkbar und unter unterschiedlichen relativen Winkeln einstellbar. Als Achse werden hier die zentralen Längsmittellinien der rohrförmigen Tubenabschnitte bezeichnet, die auch bogenförmig gekrümmt sein können und die ohne Gelenk zwischen dem proximalen und dem distalen Ende glatt und ohne Knick gegebenenfalls in einer leichten Krümmung ineinander übergehen würden, wobei das Gelenk eine relative Verschwenkung bzw. einen leichten Knick das Achsenverlaufes mit einem vergleichsweise kleine Krümmungsradius ermöglichen soll, der weniger als die Hälfte, vorzugsweise weniger als ein Fünftel des sonstigen minimalen Krümmungsradius des Tubus beträgt. Das distale Ende des Tubus kann somit gegenüber dem proximalen Ende des Tubus in verschiedene Richtungen verschwenkt werden. Während des Einführens der Trachealkanüle in die Trachea und auch danach kann sich das proximale Ende des Tubus zusammen mit dem Kehlkopf und der Trachea bewegen, ohne dass das distale Ende des Tubus gegen die Wand der Trachea drückt oder an dieser reibt. Die gelenkige Verbindung des proximalen Endes mit dem distalen Ende erhöht die Beweglichkeit der Trachealkanüle und erleichtert das Schlucken.

In einer Ausführungsform weist der Tubus auf seinem äußeren Umfang einen befüllbaren Cuff zur Abdichtung und weichen Fixierung des Tubus in der Trachea auf, wobei das Gelenk in einem Bereich angeordnet ist, der durch die äußeren distalen und proximalen Befestigungsränder des Cuffs begrenzt ist.

Bei einer solchen Ausführungsform besteht ein Cuff aus einem aufblasbaren, sehr dünnwandigen Schlauch, dessen Endabschnitte außen auf dem Tubus eng anliegen und dort befestigt sind und der mindestens in einem zentralen Abschnitt gegenüber dem Tubus ein deutliches Übermaß hat oder auf ein solches Übermaß aufblasbar ist, so dass dieser Abschnitt sich nach dem Einführen der Kanüle in die Trachea aufblasen lässt und mit der Wand der Trachea entlang seines Umfangs abdichtend und mit vorzugsweise geringem Druck in Kontakt kommt. Die Endabschnitte, die im Allgemeinen ihrerseits Schlauchabschnitte (hier auch "Halsabschnitte" genannt) darstellen, sind typischerweise über ihre Länge und ihren Umfang hinweg mit der Außenwand des Kanülenrohres (Tubus) dicht verklebt oder verschweißt. Die axial äußeren Ränder dieser Endabschnitte legen dann die Grenzen des vorstehend definierten Bereiches für eine bevorzugte Anordnung eines Gelenks fest.

In einer Ausführungsform sind das proximale und das distale Ende des jeweiligen Tubus durch einen ringförmigen Spalt mit einer lichten Spaltbreite d voneinander beabstandet und durch das axial luftdurchlässige Gelenk miteinander verbunden, welches den Spalt überbrückt.

In einer Ausführungsform befindet sich der ringförmige Spalt in einem axialen Bereich eines distalen oder proximalen Halsabschnittes eines Cuffs. D. h. der End- bzw. Halsabschnitt des Cuffs verbindet das proximale und distale Ende des jeweiligen Tubus miteinander und wirkt entweder selbst als Gelenk oder umfasst ein entsprechendes Gelenk. Die Wandstärke und Elastizität des Halsabschnittes muss gegebenenfalls an diesen Zweck angepasst sein.

Vorzugsweise ist das Gelenk mit dem proximalen Ende und/oder dem distalen Ende verklebt, verschweißt und/oder angespritzt. Damit durch die Gelenkverbindung kein Atemgas entweichen kann, ist in einer Ausführungsform vorgesehen, dass das Gelenk gasdicht mit dem proximalen Ende und dem distalen Ende des Tubus verbunden ist. Vorzugsweise ist das Gelenk radial luftundurchlässig. In einer Ausführungsform weist das distale Ende relativ zu der Achse des proximalen Endes über das Gelenk einen maximalen Biegewinkel in einem Bereich von mindestens +10° bis -10°, vorzugsweise einen Bereich von +25° bis -25°, in beliebiger Richtung relativ zur Tubusachse auf.

Zweckmäßig ist das Gelenk derart ausgestaltet, dass der Gelenkwinkel zwischen dem proximalen Ende und dem distalen Ende mit einer um mehr als 70 % verringerten Kraft F um 10° veränderbar ist, als sie aufgewendet werden muss, um das proximale Ende ohne Gelenk um 10° zu verbiegen. Ein derart ausgestaltetes Gelenk ist ausreichend flexibel um einen Schluckprozess nicht zu erschweren und dennoch ausreichend fest, um ein einfaches Einführen der Trachealbeatmungsvorrichtung in eine Trachea zu ermöglichen.

In einer Ausführungsform sind sowohl der Tubus, d. h. dessen proximales und distales Ende, als auch das Gelenk aus Kunststoff hergestellt, wobei das Gelenk aus einem Kunststoff hergestellt ist, der im Vergleich zu dem Kunststoff des Tubus (Kanülenrohrs) eine geringere Shore-Härte aufweist. Der Kunststoff des Gelenks mit der im Vergleich zu dem Kunststoff des Kanülenrohrs geringeren Shore-Härte ist weicher und daher leichter verformbar und ermöglicht auf diese Weise eine Gelenkbewegung zwischen dem proximalen und distalen Rohrabschnitt. Die im Vergleich zu dem Kunststoff des Gelenks aus einem härteren Kunststoff hergestellten Rohrabschnitte sind hinreichend fest, um einfach in die Trachea eines Patienten eingeführt zu werden. Die aus einem härteren Kunststoff hergestellten Rohrabschnitte haben vorzugsweise eine Shore-A-Härte von 50 bis 98. Der im Vergleich hierzu weichere Kunststoff des Gelenks hat eine um mindestens 50 % geringere Shore-A-Härte.

Die entsprechende Gelenkbeweglichkeit lässt sich auch mit dem gleichen Kunststoffmaterial erzielen, wie es für die proximalen und distalen Rohrabschnitte verwendet wird, das jedoch in dem vorgesehenen Gelenkbereich eine geringere Wandstärke aufweist Die Wandstärke der proximalen und distalen Rohrabschnitte beträgt vorzugweise zwischen 0,8 mm und 1,8 mm. Demgegenüber beträgt die Wandstärke des Gelenks vorzugsweise zwischen 0,1 mm bis 0,4 mm. Weist das Gelenk einen Schlauchabschnitt auf, so kann die Wandstärke des Schlauchabschnittes deutlich verringert werden. Beispielsweise kann die Wandstärke eines Schlauchabschnittes 10 µm betragen.

In einer Ausführungsform ist das Gelenk in axialer Richtung des Kanülenrohrs elastisch dehnbar und/oder stauchbar, wobei die axiale Spaltbreite d des ringförmigen Spalts zwischen dem proximalen Rohrabschnitt und dem distalen Rohrabschnitt die maximale Stauchbarkeit vorgibt. Die Spaltbreite d ist in einer Ausführungsform 1 mm bis 10 mm, vorzugsweise 2 mm bis 4 mm. Die Stauchbarkeit ist so eingestellt, dass der distale Rohrabschnitt in Richtung des proximalen Rohrabschnittes bewegt wird, wenn der distale Rohrabschnitt mit einer Kraft größer als 0,05 N auf die Trachealwand drückt. Die elastische Stauchbarkeit und Dehnbarkeit sind durch die Verwendung eines entsprechend dünnen und/oder weichen Gelenkmaterials einstellbar.

Wenn eine solches dehn- und/oder stauchbares Gelenk innerhalb eines zentralen Cuff angeordnet ist, dämpft der Cuff die Relativbewegung zwischen dem proximalen Rohrabschnitt und dem distalen Rohrabschnitt. Werden der proximale Rohrabschnitt und der distale Rohrabschnitt aufeinander zubewegt, sinkt das Cuffvolumen und der Cuffdruck steigt. Vergrößert sich der Abstand zwischen dem proximalen Rohrabschnitt und dem distalen Rohrabschnitt dehnt sich das Volumen des Cuffs aus und der Cuffdruck sinkt. Umgekehrt kann man über eine Einstellung des Cuffdrucks innerhalb gewisser Grenzen auch die Dehnung bzw. das Maß des elastischen Zusammenziehens dehnbarer elastischer Schlauchabschnitte des Gelenks einstellen.

Der Cuffdruck ermöglicht eine Pufferung des Stauchens der Kanüle in axialer Richtung des Kanülenrohrs. Das Gelenk und der das Gelenk umgebende, unter leichtem Überdruck mit Gas befüllte Cuff wirken wie ein Stoßdämpfer, der Stöße auf die Trachealbeatmungsvorrichtung aufnimmt und verringert. Auf diese Weise können Schädigungen der Trachealwand durch das distale Kanülenende reduziert und der Schluckvorgang erleichtert werden. Vorzugsweise wird ein Cuff mit Hilfe von Luft mit einem Druck von 15 mbar bis 30 mbar beaufschlagt. Der radial außenliegend zu dem ringförmigen Spalt angeordnete Cuff bewirkt eine Rückstellkraft in die Ausgangslage des Gelenks. Zugleich verhindert der befüllte Cuff, dass die an dem Spalt befindlichen Enden des proximalen und des distalen Rohrabschnittes gegen die Trachea drücken und diese schädigen. Der Cuff polstert den ringförmigen Spalt und das Gelenk der Trachealbeatmungsvorrichtung gegenüber der Trachea ab, so dass eine bündige Fixierung eines das Gelenk bildenden Schlauchabschnittes an den jeweiligen Rohrabschnitten nicht erforderlich ist.

In einer Ausführungsform ist das Gelenk ein Schlauchabschnitt, der auf der einen Seite eines ringförmigen Spalts zwischen dem proximalen und dem distalen Rohrabschnitt an dem proximalen Rohrabschnitt und auf der anderen Seite des Spalts an dem distalen Rohrabschnitt befestigt ist. Vorzugsweise weist der Kunststoff des Schlauchabschnitts eine im Vergleich zu dem Kunststoff des Kanülenrohrs geringere Shore-Härte auf. Der Schlauchabschnitt aus dem weicheren Kunststoff ist elastisch verformbar, sodass die Spaltbreite d, d.h. der lichte Abstand zwischen dem proximalen Rohrabschnitt und dem distalen Rohrabschnitt, auf gegenüberliegenden Seiten der Rohrabschnitte in unterschiedlichem Maße und/oder in unterschiedlicher Richtung veränderbar ist. Wird der Schlauchabschnitt einseitig gestaucht oder gedehnt, verkleinert oder vergrößert sich die Spaltbreite d des ringförmigen Spalts entsprechend auf einer Seite, sodass der proximale Rohrabschnitt und der distale Rohrabschnitt relativ zueinander verschwenkt werden.

In einer Ausführungsform ist der Schlauchabschnitt beidseitig des ringförmigen Spalts an dem proximalen Rohrabschnitt und dem distalen Rohrabschnitt befestigt. Vorzugsweise ist der Schlauchabschnitt stumpf auf die Stirnseiten des proximalen und des distalen Rohrabschnittes aufgesetzt verklebt, verschweißt oder angespritzt. In einer anderen Ausführungsform weisen die einander zugewandten Enden der Rohrabschnitte einen Falz zur bündigen und glatten Aufnahme des komplementär ausgestalten Schlauchabschnittes auf. Der Schlauchabschnitt verbindet wie eine Muffe den proximalen Rohrabschnitt und den distalen Rohrabschnitt für die Durchleitung von Atemgas.

In einer Ausführungsform weist das Gelenk zusätzlich zu dem zuvor beschriebenen Schlauchabschnitt einen zweiten Schlauchabschnitt auf, der konzentrisch zu dem ersten Schlauchabschnitt angeordnet ist, wobei der erste Schlauchabschnitt radial außenliegend mit dem proximalen Rohrabschnitt und dem distalen Rohrabschnitt verbunden und der zweite Schlauchabschnitt radial innenliegend mit dem proximalen Rohrabschnitt und dem distalen Rohrabschnitt verbunden ist. Die zwei Schlauchabschnitte verbinden den proximalen Rohrabschnitt und den distalen Rohrabschnitt sowohl auf der Innenseite des Kanülenrohrs, d.h. im zentralen Lumen des Kanülenrohrs, als auch auf der Außenseite des Kanülenrohrs. Der erste Schlauchabschnitt und/oder der zweite Schlauchabschnitt überspannen den ringförmigen Spalt und sind beidseitig des ringförmigen Spalts an dem proximalen Rohrabschnitt und dem distalen Rohrabschnitt befestigt. Beispielsweise ist der eine oder sind beide Schlauchabschnitte durch Verkleben, Verschweißen oder Anspritzen an den proximalen und distalen Rohrabschnitten befestigt.

Vorzugsweise weist der eine Schlauchabschnitt des Gelenks, oder falls das Gelenk aus zwei Schlauchabschnitten besteht, beide Schlauchabschnitte, eine geringere Wandstärke als die mittlere Wandstärke des proximalen Rohrabschnitts im Anschlussbereich des Gelenks auf. Beispielsweise ist die mittlere Wandstärke des ersten Schlauchabschnittes und/oder des zweiten Schlauchabschnittes ¼ der mittleren Wandstärke des proximalen Rohrabschnitts im Anschlussbereich des Gelenks.

In einer Ausführungsform ist der Schlauchabschnitt stumpf mit den Enden der proximalen und distalen Rohrabschnitte verbunden. Somit füllt der Schlauchabschnitt den ringförmigen Spalt zwischen dem proximalen Rohrabschnitt und dem distalen Rohrabschnitt aus. Der Schlauchabschnitt kann sich nahtlos an den proximalen Rohrabschnitt und den distalen Rohrabschnitt anschließen. Der Schlauchabschnitt hat in seinem proximalen Anschlussbereich eine Wandstärke, die der Wandstärke des proximalen Rohrabschnittes entspricht, und in seinem distalen Anschlussbereich eine Wandstärke, die der Wandstärke des distalen Rohrabschnittes entspricht. In einer etwas modifizierten Ausführungsform hat der der Schlauchabschnitt eine größere Wandstärke als der proximale Rohrabschnitt und der distale Rohrabschnitt, mit je einer ringförmigen Nut in den Stirnseiten des Schlauchabschnittes für die Aufnahme der Enden des proximalen und distalen Rohrabschnitte in diesen Nuten. Die sich über den Spalt in axialer Länge des Kanülenrohrs hinaus und in den Bereich des proximalen und distalen Rohabschnittes erstreckenden Abschnitte des Schlauchabschnittes sind beidseitig des Spaltes an dem proximalen Rohrabschnitt und dem distalen Rohrabschnitt befestigt, vorzugsweise verklebt, verschweißt oder angespritzt.

Um das Gelenk zu stabilisieren, weist das Gelenk in einer Ausführungsform eine Mehrzahl von Kunststoffstegen, vorzugsweise zwei diametral gegenüberliegend angeordnete Kunststoffstege auf, die den ringförmigen Spalt überbrücken und den proximalen Rohrabschnitt mit dem distalen Rohrabschnitt verbinden. Vorzugsweise sind die Kunststoffstege gleichmäßig in Umfangsrichtung des ringförmigen Spalts verteilt angeordnet. Die Anordnung der Kunststoffstege ist geeignet, die Freiheitsgrade des Gelenks einzuschränken. Weist das Gelenk beispielsweise zwei Kunststoffstege auf, die in einem Winkelabstand von 180° oder auch etwas weniger in Umfangsrichtung des ringförmigen Spalts angeordnet sind, weist das Gelenk, ähnlich einem Scharniergelenk, eine bevorzugte Schwenkrichtung in etwa parallel zu den Kunststoffstegen auf. In einer Ausführungsform sind die Stege seitlich angeordnet, sodass mit dem Gelenk der Biegewinkel des Kanülenrohrs vergrößert oder verkleinert werden kann, zusätzlich eine Verbiegbbarkeit in seitliche Richtung reduziert wird. Die Kunststoffstege können aus dem gleichen Kunststoff wie die Rohrabschnitte oder aus einem im Vergleich hierzu weicheren Kunststoff gefertigt sein. Gleichzeitig ist das Gelenk durch die Stege gegenüber axialen Kräften versteift.

In einer Ausführungsform ist die axiale Spaltbreite d des ringförmigen Spalts 1 mm bis 10 mm, vorzugsweise 3 mm bis 4 mm. Derartige Spaltbreiten verhindern, dass sich bei einer maximalen Gelenkbewegung das zentrale Lumen des Kanülenrohrs verschließt und die Atmung durch die Trachealbeatmungsvorrichtung wesentlich erschwert wird.

In einer Ausführungsform wird das Gelenk aus einem Wandabschnitt des Kanülenrohres gebildet der den proximalen Rohrabschnitt vom distalen Rohrabschnitt trennt, wobei dieser Wandabschnitt eine reduzierte Wandstärke aufweist.

Hinsichtlich der Vorrichtung zur Atemluftzuführung die aus zwei ineinander gesteckten Trachealkanülen der vorstehend beschriebenen Art besteht, wird die erfindungsgemäße Aufgabe dadurch gelöst, dass die erste Trachealkanüle als Außenkanüle und die zweite Trachealkanüle als Innenkanüle fungiert und wobei die Innenkanüle axial länger ist als die Außenkanüle so dass eine am distalen Ende der Innenkanüle angeordnete Ringmanschette distal außerhalb des distalen Endes der Außenkanüle liegt. Beide Kanülen haben dann unabhängig voneinander wirkende Abdichteinrichtungen in Form von Ringmanschetten mit Dichtrand und/oder einer entrollbaren Dichtlippe.

Eine der beiden Trachealkanülen, vorzugsweise die Innenkanüle, könnte anstelle der Ringmanschette und/oder einer entrollbaren Dichtlippe einen herkömmlichen, aufblasbaren Cuff aufweisen.

In jedem Fall sind die beiden Trachealkanülen so aufeinander abgestimmt, dass die Innenkanüle in die Außenkanüle einführbar und Hindurchführbar ist, bzw. die Außenkanüle von der Innenkanüle abgezogen werden kann. Die Innenkanüle sollte dabei einen für die Beatmung ausreichenden freien Innenquerschnitt haben. Die Innenkanüle und/oder die Außenkanüle könnte, wie in einer Ausführungsform vorgesehen, ein Gelenk im Sinne der vorliegenden Erfindung aufweisen. Weist die Innenkanüle ein Gelenk auf, so ist dieses vorzugsweise in einem Bereich angeordnet, der aus der distalen Öffnung der Außenkanüle hervorsteht.

In einer Ausführungsform einer Vorrichtung zur Atemluftzuführung mit einer ersten Trachealkanüle und einer zweiten Trachealkanüle, wobei jede Trachealkanüle einen Tubus mit einem proximalen Ende mit einer proximalen Öffnung und ein distales Ende mit einer distalen Öffnung aufweist, weist die erste Trachealkanüle eine elastische Ringmanschette wie vorstehend beschrieben auf, wobei die Trachealkanüle mit der elastischen Ringmanschette derart bemessen und in die zweite Trachealkanüle einbringbar ist, dass die Ringmanschette über das distale Ende der zweiten Trachealkanüle übersteht.

In einer alternativen Ausführungsform einer Vorrichtung zur Atemluftzuführung mit einer ersten Trachealkanüle und einer zweiten Trachealkanüle, wobei jede Trachealkanüle einen Tubus mit einem proximalen Ende mit einer proximalen Öffnung und mit einem distalen Ende mit einer distalen Öffnung aufweist, weist die erste Trachealkanüle eine elastische Ringmanschette wie vorstehend beschrieben auf, wobei die zweite Trachealkanüle derart bemessen und in die erste Trachealkanüle mit der elastischen Ringmanschette einbringbar ist, dass ein auf der Außenseite des Tubus der zweiten Trachealkanüle angeordneter Cuff über das distale Ende des Tubus der ersten Trachealkanüle mit der elastischen Ringmanschette übersteht.

Soweit in der vorliegenden Beschreibung, einschließlich der nachfolgenden Figurenbeschreibung Aspekte der Erfindung im Hinblick auf die Trachealkanüle mit einer Ringmanschette beschrieben werden, gelten diese Merkmale auch für die erste Trachealkanüle (Außenkanüle) und optional auch für die zweite Trachealkanüle (Innenkanüle) der erfindungsgemäßen Vorrichtungen zur Atemluftzuführung offenbart und umgekehrt.

Weitere Vorteile, Merkmale und Anwendungsmöglichkeiten der vorliegenden Erfindung werden anhand der folgenden Beschreibung von Ausführungsformen und den dazugehörigen Figuren deutlich, wobei gleiche Bezugszeichen auf gleiche Elemente verweisen. Es zeigen:
- Figur 1:: einen schematischen Längsschnitt durch eine Trachealkanüle gemäß einer Ausführungsform der Erfindung,
- Figur 2:: einen schematischen Querschnitt durch eine Trachealkanüle mit Ringmanschette und Dichtlippe aus Figur 1,
- Figur 3:: eine perspektivische Ansicht der Trachealkanüle aus Figur 1,
- Figur 4:: eine perspektivische Ansicht auf eine erfindungsgemäße Vorrichtung zur Atemluftzuführung, und
- Figur 5:: eine perspektivische Ansicht auf eine Vorrichtung zur Atemluftzuführung gemäß einer weiteren Ausführungsform der vorliegenden Erfindung.

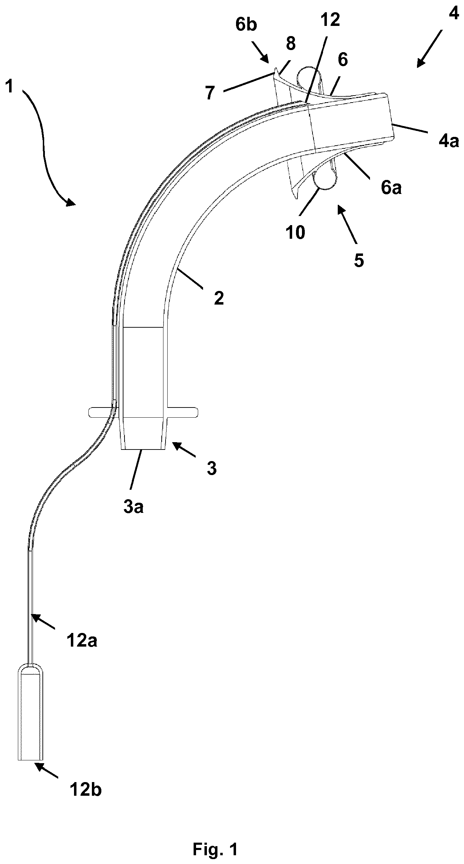

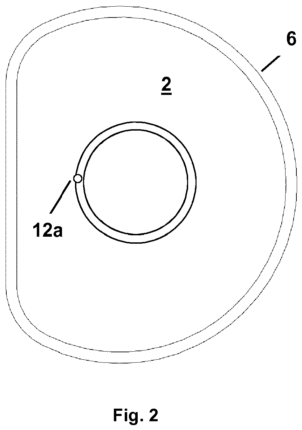

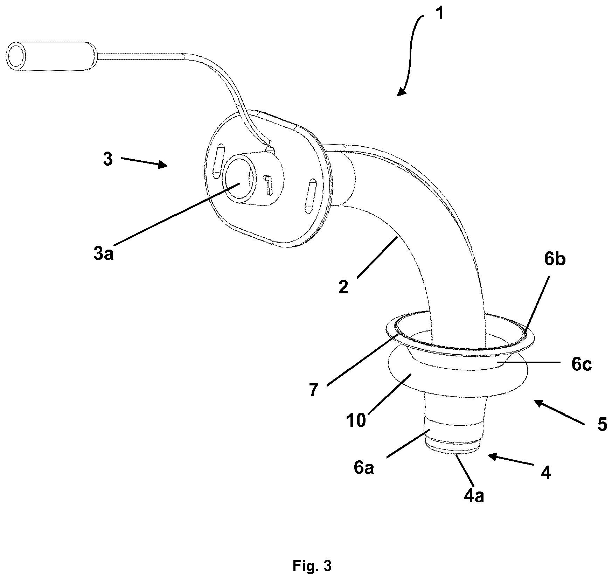

In der Figuren 1 bis 3 ist eine Trachealkanüle 1 gemäß einer Ausführungsform der vorliegenden Erfindung dargestellt, wobei die Figur 1 einen Längsschnitt, Figur 2 einen Querschnitt und Figur 3 eine Perspektivansicht zeigen. Die Trachealkanüle 1 hat einen bogenförmig gekrümmten Tubus 2 mit einem proximalen Ende 3 und einem distalen Ende 4. Das proximale Ende 3 weist eine proximale Öffnung 3a und das distale Ende 4 eine distale Öffnung 4a auf. Durch den Tubus 2 kann Atemgas geführt werden, wobei das distale Ende 4 durch ein Tracheostoma in die Trachea eines Patienten einführbar ist, während das proximale Ende 3 außerhalb des Patienten mit einer Beatmungsvorrichtung verbindbar ist.

Damit Sekret aus dem subglottischen Raum der Trachea nicht an dem Tubus 2 vorbei in die Bronchien eines Patienten gelangen kann, weist der Tubus 2 an seinem äußeren Umfang nahe seines distalen Endes 4 eine Abdichteinrichtung 5 auf. Erfindungsgemäß ist die Abdichteinrichtung 5 eine ringförmig umlaufende und trichterförmig nach außen und zum proximalen Ende 3 hin gewölbte Ringmanschette 6 mit einem distalen Ende 6a und einem proximalen Ende 6b. Das distale Ende 6a der Ringmanschette 6 umfasst den Tubus 2 und dichtet die Ringmanschette 6 gegenüber dem Tubus 2 ab. Das distale Ende 6a ist bei dieser Ausführungsform an die Außenseite des Tubus 2 angespritzt.

Das freie, proximale Ende 6b der Ringmanschette 6 ist als umlaufender, elastisch nachgiebiger Dichtrand 7 ausgebildet. Der Dichtrand 7 ist in einem Zustand, in dem das distale Ende 4 des Tubus 2 in die Trachea eines Patienten eingeführt ist, an die Innenwand der Trachea anlegbar. Der äußere Umfang des Dichtrandes 7 definiert eine Querschnittsform, die der typischen Querschnittsform einer Trachea nachempfunden ist. Auf diese Weise kann sich der Dichtrand 7 leicht entlang seines gesamten Umfangs dicht an die Innenwand der Trachea anlegen.

Um die dichte Anlage zu gewährleisten, ist der Dichtrand 7 bei einer in der Trachea befindlichen Trachealkanüle radial nach außen vorgespannt. Der Dichtrand 7 weist an seinem radial innenliegenden Rand einen Versteifungsabschnitt 8 auf, der umlaufend entlang des radial inneren Randes angeordnet ist. Beim Einführen der Trachealkanüle 1 in eine Trachea streicht der umlaufende Dichtrand 7 über die Innenseite der Trachea. Beim Herausziehen der Trachealkanüle 1 aus der Trachea kann der radial umlaufende Dichtrand 7 in Richtung des distalen Endes 4 des Tubus 2 umklappen, was das Herausziehen erleichtert.

In einem an die Trachea angelegten Zustand verhindert der Dichtrand 7, dass Sekret aus dem subglottischen Raum an dem Tubus 2 vorbei in die Bronchien gelangt. Das Sekret gelangt stattdessen über den Dichtrand 7 in den Zwischenraum zwischen der trichterförmig aufgeweiteten Ringmanschette 6 und dem Tubus 2. Das die Ringmanschette 6 gegenüber dem Tubus 2 abdichtende, distale Ende 6a verhindert, dass das Sekret weiter in die distale Richtung fließen kann. Das Sekret wird in dem Zwischenraum zwischen der Ringmanschette 6 und dem Tubus 2 gesammelt. Um das Sekret aus dem Zwischenraum zu entfernen, weist der Tubus 2 auf seiner Außenseite eine Spül- und/oder Absaugöffnung 12 auf, die in dem Bereich zwischen dem proximalen Ende 6b und dem distalen Ende 6a der Ringmanschette 6 angeordnet ist. Die Spül- und/oder Absaugöffnung 12 ist über einen in das Material des Tubus 2 eingelassenen Spülkanal 12a mit einem Konnektor zum Anschluss an eine Spül- und/oder Absaugvorrichtung 12b, wie beispielsweise eine Spritze, verbunden.

Zwischen ihrem proximalen Ende 6b und ihrem distalen Ende 6a weist die Ringmanschette 6 einen Übergangsabschnitt 6c auf, der nach Art einer Trompetenöffnung von innen nach außen gekrümmt ist. Auf der Außenseite des Übergangsabschnittes 6c setzt an der Ringmanschette 6 eine eingerollte Dichtlippe 10 an. Die Dichtlippe 10 erstreckt sich in ihrem eingerollten Zustand in einer Querschnittsansicht kreis oder spiralförmig über einen Winkelbogen von mehr als 180° aber weniger als 270°. Die in der Querschnittsform durch den nicht geschlossenen Winkelbogen gebildete Öffnung der eingerollten Dichtlippe 10 zeigt in die distale Richtung. Trifft ein Luftstrom aus der distalen Richtung auf die eingerollte Dichtlippe 10, wird sie entrollt und aufgeweitet. Der radial äußere Bereich der Dichtlippe wird gegen die Innenwand der Trachea gedrückt und verhindert, dass der Luftstrom weiter in die proximale Richtung fließen kann.

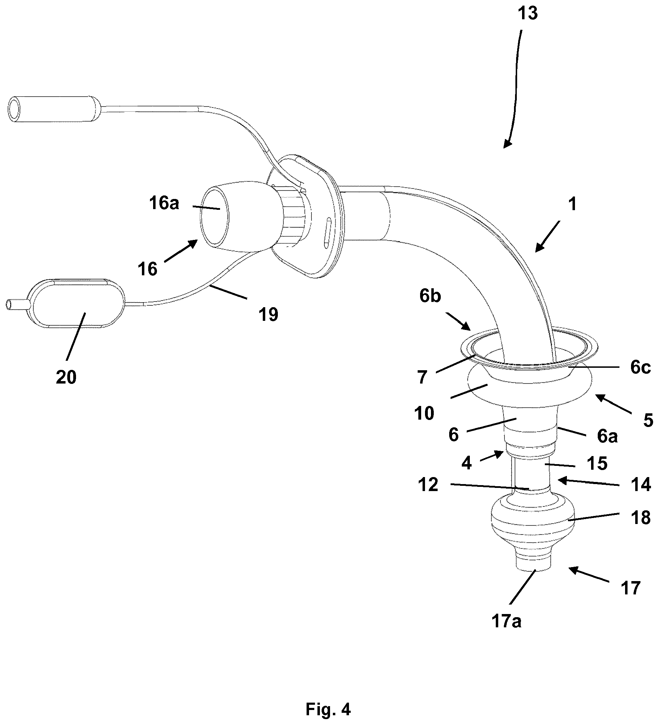

In Figur 4 ist eine Vorrichtung zur Atemluftzuführung 13 gemäß einer Ausführungsform der Erfindung dargestellt. Die Vorrichtung zur Atemluftzuführung 13 umfasst eine Trachealkanüle 1 wie sie zuvor im Zusammenhang mit den Figuren 1 bis 3 beschrieben wurde und eine zweite Trachealkanüle 14. Die zweite Trachealkanüle 14 hat, genau wie die erste Trachealkanüle 1, einen Tubus 15 mit einem proximalen Ende 16 und einem distalen Ende 17. Das proximale Ende 16 weist eine proximale Öffnung 16a und das distale Ende 17 eine distale Öffnung 17a auf.

Die zweite Trachealkanüle 14 ist derart bemessen, dass sie in die erste Trachealkanüle 1 mit der Ringmanschette 6 einbringbar ist und ein auf der Außenseite an dem distalen Ende 17 der zweiten Trachealkanüle 14 angeordneter, Cuff 18 vollständig freiliegend über das distalen Ende 4 der Trachealkanüle 1 mit der Ringmanschette 6 übersteht.

Der Cuff 18 der zweiten Trachealkanüle 14 ist über einen Füllschlauch 19 mit einer Füllvorrichtung 20 verbunden. Das Innere des Cuffs 18 der zweiten Trachealkanüle 14 kann so mit einem Fluid beaufschlagt und auf diese Weise entfaltet und aufgebläht werden. Der Cuff 18 ist dadurch an die Innenwand einer Trachea dicht anlegbar und hilft so, die Trachealkanüle 18 gegenüber der Trachea abzudichten.

Proximal von dem Cuff 18 und distal zu dem distalen Ende 4 der Trachealkanüle 1 mit der Ringmanschette 6 weist die zweite Trachealkanüle 14 eine Spül- und/oder Absaugöffnung 12 auf. Die Spül- und/oder Absaugvorrichtung 12 steht in dem in der Figur 4 gezeigten Zustand, in dem die zweite Trachealkanüle 14 in den Tubus 2 der Trachealkanüle 1 mit der Ringmanschette 6 eingebracht ist, freiliegend über das distalen Ende 4 der Trachealkanüle 1 mit der Ringmanschette 6 über.

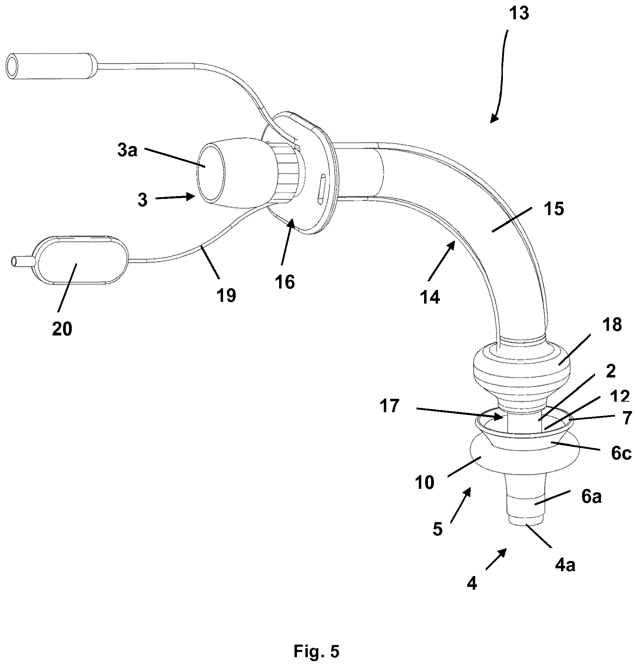

Eine weitere Ausführungsform einer erfindungsgemäßen Vorrichtung zur Atemluftzuführung 13 ist in der Figur 5 gezeigt. Genau wie die Vorrichtung zur Atemluftzuführung 13 aus Figur 4 umfasst die Vorrichtung zur Atemluftzuführung 13 der Figur 5 eine Trachealkanüle 1 entsprechend der in den Figuren 1 bis 3 gezeigten Ausführungsform sowie eine zweite Trachealkanüle 14.

Die zweite Trachealkanüle 14 hat einen Tubus 15 mit einem proximalen Ende 16 und einem distalen Ende 17. Das proximale Ende 16 weist eine proximale Öffnung 16a und das distale Ende 17 eine distale Öffnung 17a auf. Anders als bei der Ausführungsform gemäß der Figur 4, ist bei der in der Figur 5 gezeigten Ausführungsform die Trachealkanüle 1 mit der Ringmanschette 2 derart bemessen und in die zweite Trachealkanüle 14 einbringbar, dass die Ringmanschette 6 über das distale Ende 17 der zweiten Trachealkanüle 14 übersteht.

Der Tubus 15 der zweiten Trachealkanüle 14 weist auf seiner Außenseite einen Cuff 18 auf. Das Innere des Cuffs 18 ist über einen Füllschlauch 19 und eine Füllvorrichtung 20 mit einem Fluid beaufschlagbar, sodass der Cuff 18 in seinem entfalteten Zustand an die Innenwand einer Trachea anlegbar ist. Der Cuff 18 hilft die zweite Trachealkanüle 14 gegenüber einer Trachea abzudichten. Die Spül- und/oder Absaugöffnung 12 der Trachealkanüle 1 mit der Ringmanschette 6 steht freiliegend über das distale Ende 17 des Tubus 15 der zweiten Trachealkanüle 14 über. Somit kann Sekret, das am Cuff 18 vorbeifließt aufgefangen und abgesaugt werden. In der Perspektivansicht der Figur 5 ist dies nicht gezeigt. Die Absaugöffnung entspricht der in der Figur 1 gezeigten Absaugöffnung.

Für Zwecke der ursprünglichen Offenbarung wird darauf hingewiesen, dass sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarerer Merkmalskombinationen wird hier nur der Kürze und der Lesbarkeit der Beschreibung wegen verzichtet.

Während die Erfindung im Detail in den Zeichnungen und der vorangehenden Beschreibung dargestellt und beschreiben wurde, erfolgt diese Darstellung und Beschreibung lediglich beispielhaft und ist nicht als Beschränkung des Schutzbereichs gedacht, so wie er durch die Ansprüche definiert wird. Die Erfindung ist nicht auf die offenbarten Ausführungsformen beschränkt.

Abwandlungen der offenbarten Ausführungsformen sind für den Fachmann aus den Zeichnungen, der Beschreibung und den beigefügten Ansprüchen offensichtlich. In den Ansprüchen schließt das Wort "aufweisen" nicht andere Elemente oder Schritte aus, und der unbestimmte Artikel "eine" oder "ein" schließt eine Mehrzahl nicht aus. Die bloße Tatsache, dass bestimmte Merkmale in unterschiedlichen Ansprüchen beansprucht sind, schließt ihre Kombination nicht aus. Bezugszeichen in den Ansprüchen sind nicht als Beschränkung des Schutzbereichs gedacht.

### Bezugszeichenliste

- 1: Trachealkanüle
- 2: Tubus
- 3: proximales Ende des Tubus 2
- 3a: proximale Öffnung
- 4: distales Ende des Tubus 2
- 4a: distale Öffnung
- 5: Abdichteinrichtung
- 6: Ringmanschette
- 6a: distales Ende der Ringmanschette 6
- 6b: proximales Ende der Ringmanschette 6
- 6c: Übergangsabschnitt
- 7: Dichtrand
- 8: Versteifungsabschnitt
- 9: Versteifungsrippen
- 10: eingerollte Dichtlippe
- 11: Durchbrechungen
- 12: Spül- und/oder Absaugöffnung
- 12a: Spülkanal
- 12b: Konnektor zum Anschluss an eine Spül- und/oder Absaugvorrichtung
- 13: Vorrichtung zur Atemluftzuführung
- 14: zweite Trachealkanüle
- 15: Tubus
- 16: proximales Ende des Tubus 15
- 16a: proximale Öffnung
- 17: distales Ende des Tubus 15
- 17a: distale Öffnung
- 18: Cuff
- 19: Füllschlauch
- 20: Füllvorrichtung

## Patentansprüche

1. Trachealkanüle (1), insbesondere Tracheostomiekanüle oder Endotrachealtubus, mit einem Tubus (2), wobei der Tubus (2) ein proximales Ende (3) mit einer proximalen Öffnung (3a), ein distales Ende (4) mit einer distalen Öffnung (4a) und eine an seinem äußeren Umfang nahe des distalen Ende (4) angeordneten Abdichteinrichtung (5) aufweist, wobei die Abdichteinrichtung (5) eine ringförmig umlaufende und trichterförmig nach außen und zum proximalen Ende (3) gewölbte Ringmanschette (6) ist, deren distales Ende (6a) den Tubus (2) dicht umfasst und deren freies, proximales Ende (6b) als umlaufender, elastisch nachgiebiger Dichtrand (7) ausgebildet ist **dadurch gekennzeichnet, dass** die Ringmanschette (6) distal unterhalb ihres proximalen Endes zusätzlich zu dem proximal darüber befindlichen Dichtrand (7) eine umlaufende, in Richtung des distalen Endes der Ringmanschette (6) eingerollte Dichtlippe (10) aufweist.

2. Trachealkanüle (1) nach Anspruch 1, **dadurch gekennzeichnet, dass** die trichterförmige Ringmanschette (6) elastisch verformbar ist, wobei der elastische Dichtrand (7) radial nach außen und/oder in Umfangsrichtung vorgespannt ist.

3. Trachealkanüle (1) nach Anspruch 1 oder 2, **dadurch gekennzeichnet, dass** der elastische Dichtrand (7) zumindest einen Versteifungsabschnitt (8) aufweist, wobei sich zumindest ein Versteifungsabschnitt (8) entlang des radial innenliegenden Randes des Dichtrandes (7) und/oder ein Versteifungsabschnitt (8) parallel zu und im Abstand zu dem radial außenliegenden Rand des Dichtrandes (7) erstreckt.

4. Trachealkanüle (1) nach einem der Ansprüche 1 bis 3, **dadurch gekennzeichnet, dass** der elastische Dichtrand (7) zumindest einen Versteifungsabschnitt (8) in Form einer Vielzahl sich radial erstreckender Versteifungsrippen (9) aufweist, und/oder der elastische Dichtrand (7) entweder die Form einer Dichtlippe hat oder ein vorzugsweise torusförmiger Dichtwulst aus einem weichelastischen Material ist.

5. Trachealkanüle (1) nach einem der Ansprüche 1 bis 4, **dadurch gekennzeichnet, dass** der elastische Dichtrand (7) eine Shore-A-Härte von 3 bis 60, vorzugsweise 10 bis 40, aufweist.

6. Trachealkanüle (1) nach einem der Ansprüche 1 bis 5, **dadurch gekennzeichnet, dass** der radial äußere Umfang des Dichtrandes (7) eine kreisförmige Kontur, eine ovale Kontur, oder eine D-förmige Kontur mit abgerundeten Ecken, insbesondere eine an einen typischen Tracheaquerschnitt angenäherte Kontur aufweist.

7. Trachealkanüle (1) nach einem der Ansprüche 1 bis 6, **dadurch gekennzeichnet, dass** das distale Ende (6a) der Ringmanschette (6) unmittelbar und dicht in das Material des Tubus (2) übergeht und/oder die Ringmanschette (6) zwischen ihrem proximalen Ende (6b) und ihrem distalen Ende (6a) einen in Längsrichtung der Ringmanschette (6) gekrümmten Übergangsabschnitt (6c) aufweist.

8. Trachealkanüle (1) nach einem der Ansprüche 1 bis 7, **dadurch gekennzeichnet, dass** die Materialstärke der Dichtlippe (10) derart bemessen ist, dass sich der Querschnitt der Dichtlippe (10) bei Auftreffen eines sich von distal nach proximal bewegenden Fluidstroms mit einem Druck ≥ 3 mbar, vorzugsweise einem Druck ≥ 15 mbar, radial nach außen aufweitet.

9. Trachealkanüle (1) nach Anspruch 7 oder 8, **dadurch gekennzeichnet, dass** die eingerollte Dichtlippe (10) sich in ihrem eingerollten Zustand in einer Querschnittsansicht näherungsweise entlang eines Kreis- oder Spiralbogens von weniger als 360°, vorzugsweise weniger als 300° oder weniger als 270°, aber mehr als 180°, vorzugsweise mehr als 200° und besonders bevorzugt mehr als 225° erstreckt und/oder die Ringmanschette (6) proximal zu der Dichtlippe (10) eine Mehrzahl von Durchbrechungen (11) in der Ringmanschettenwand aufweist, wobei die Durchbrechungen (11) vorzugsweise einen Durchmesser in einem Bereich von 0,2 mm bis 2 mm, vorzugsweise in einem Bereich von 0,5 mm bis 1,5 mm, aufweisen.

10. Trachealkanüle (1) nach einem der vorstehenden Ansprüche, **dadurch gekennzeichnet, dass** die Weichheit der eingerollten Dichtlippe (10) und/oder die Weichheit des Dichtrandes (7) derart ausgestaltet ist, dass die eingerollte Dichtlippe (10) und/oder der Dichtrand (7) bei einem expiratorischen Druck ≥ 30 mbar in proximale Richtung umklappt und bei einem solchen Druck in dieser Position verbleibt.

11. Trachealkanüle (1) nach einem der Ansprüche 1 bis 10, **dadurch gekennzeichnet, dass** der Tubus (2) auf seiner Außenseite eine Spül- und/oder Absaugöffnung (12) in einem Bereich zwischen dem proximalen Ende (6b) der Ringmanschette (6) und dem distalen Ende (6a) der Ringmanschette (6) aufweist, wobei die Spül- und/oder Absaugöffnung (12) mit einer Spül- und/oder Absaugvorrichtung verbindbar ist.

12. Trachealkanüle (1) nach einem der Ansprüche 1 bis 11, **dadurch gekennzeichnet, dass** der Tubus (2) auf seiner Außenseite eine Spül- und/oder Absaugöffnung (12) in einem Bereich zwischen dem distalen Ende (6b) der Ringmanschette (6) und der eingerollten Dichtlippe aufweist, wobei die Spül- und/oder Absaugöffnung (12) mit einer Spül- und/oder Absaugvorrichtung verbindbar ist.

13. Trachealkanüle (1) nach einem der Ansprüche 1 bis 12, **dadurch gekennzeichnet, dass** die Trachealkanüle (1) ein axial luftdurchlässiges Gelenk aufweist, welches das proximale Ende (3) und das distale Ende (4) des Tubus (2) miteinander verbindet und mindestens eine begrenzte Beweglichkeit und insbesondere Verschwenkbarkeit der Achse des distalen Endes (4) bezüglich der Achse des proximalen Endes (3) ermöglicht.

14. Vorrichtung zur Atemluftzuführung (13) mit einer ersten Trachealkanüle (1) und einer zweiten Trachealkanüle (14) mit einem Tubus (15), wobei der Tubus (15) ein proximales Ende (16) mit einer proximalen Öffnung (16a), ein distales Ende (17) mit einer distalen Öffnung (17a) aufweist, **dadurch gekennzeichnet, dass** die erste Trachealkanüle (1) nach einem der Ansprüche 1 bis 13 ausgestaltet ist, wobei die Trachealkanüle (1) mit der elastischen Ringmanschette (6) derart bemessen und in die zweite Trachealkanüle (14) einbringbar ist, dass die Ringmanschette (6) über das distale Ende (17) der zweiten Trachealkanüle (14) übersteht.

15. Vorrichtung zur Atemluftzuführung (13) mit einer ersten Trachealkanüle (1) und einer zweiten Trachealkanüle (14) mit einem Tubus (15), wobei der Tubus (15) ein proximales Ende (16) mit einer proximalen Öffnung (16a), ein distales Ende (17) mit einer distalen Öffnung (17a) aufweist, **dadurch gekennzeichnet, dass** die erste Trachealkanüle (1) nach einem der Ansprüche 1 bis 13 ausgestaltet ist, wobei die zweite Trachealkanüle (14) derart bemessen und in die erste Trachealkanüle (1) mit der elastischen Ringmanschette (6) einbringbar ist, dass ein auf der Außenseite des Tubus (15) der zweiten Trachealkanüle (14) angeordneter Cuff (18) über das distale Ende (17) des Tubus (2) der ersten Trachealkanüle (1) mit der elastischen Ringmanschette (6) übersteht.

## Claims

1. A tracheal cannula (1), in particular a tracheostomy cannula or endotracheal tube, comprising a tube (2), wherein the tube (2) has a proximal end (3) with a proximal opening (3a), a distal end (4) with a distal opening (4a) and a sealing device (5) arranged at its outer periphery near the distal end (4), wherein the sealing device (5) has an annularly peripherally extending ring cuff (6) which is curved in a funnel configuration outwardly and towards the proximal end (3) and whose distal end (6a) sealingly surrounds the tube (2) and whose free proximal end is in the form of a peripherally extending, elastically yielding sealing edge (7), **characterised in that** the ring cuff (6) distally beneath its proximal end in addition to the sealing edge (7) disposed proximally thereabove has a peripherally extending sealing lip (10) which is rolled in in the direction of the distal end of the ring cuff (6).

2. A tracheal cannula (1) according to claim 1 **characterised in that** the funnel-shaped ring cuff (6) is elastically deformable, wherein the elastic sealing edge (7) is prestressed radially outwardly and/or in the peripheral direction.

3. A tracheal cannula (1) according to claim 1 or claim 2 **characterised in that** the elastic sealing edge (7) has at least one reinforcing portion (8), wherein at least one reinforcing portion (8) extends along the radially inwardly disposed edge of the sealing edge (7) and/or a reinforcing portion (8) extends parallel to and at a spacing from the radially outwardly disposed edge of the sealing edge (7).

4. A tracheal cannula (1) according to one of claims 1 to 3 **characterised in that** the elastic sealing edge (7) has at least one reinforcing portion (8) in the form of a plurality of radially extending reinforcing ribs (9) and/or the elastic sealing edge (7) is either in the form of a sealing lip or it is a preferably toroidal sealing bead comprising a soft-elastic material.

5. A tracheal cannula (1) according to one of claims 1 to 4 **characterised in that** the elastic sealing edge (7) has a Shore A hardness of 3 to 60, preferably 10 to 40.

6. A tracheal cannula (1) according to one of claims 1 to 5 **characterised in that** the radially outer periphery of the sealing edge (7) has a circular contour, an oval contour or a D-shaped contour with rounded corners, in particular a contour approximated to a typical trachea cross-section.

7. A tracheal cannula (1) according to one of claims 1 to 6 **characterised in that** the distal end (6a) of the ring cuff (6) transitions directly and sealingly into the material of the tube (2) and/or the ring cuff (6) has a transitional portion (6c) curved in the longitudinal direction of the ring cuff (6) between the proximal end (6b) and the distal end (6a) of the ring cuff.

8. A tracheal cannula (1) according to one of claims 1 to 7 **characterised in that** the material thickness of the sealing lip (10) is such that the cross-section of the sealing lip (10) enlarges radially outwardly upon impact of a fluid flow moving from distal towards proximal at a pressure ≥ 3 mbar, preferably a pressure ≥ 15 mbar.

9. A tracheal cannula (1) according to claim 7 or claim 8 **characterised in that** the rolled-in sealing lip (10) in its rolled-in state in a cross-sectional view extends approximately along a circular or spiral arc of less than 360°, preferably less than 300° or less than 270° but more than 180°, preferably more than 200° and particularly preferably more than 225° and/or the ring cuff (6) proximally relative to the sealing lip (10) has a plurality of through openings (11) in the ring cuff wall, wherein the through openings (11) are preferably of a diameter in a range of 0.2 mm to 2 mm, preferably in a range of 0.5 mm to 1.5 mm.

10. A tracheal cannula (1) according to one of the preceding claims **characterised in that** the softness of the rolled-in sealing lip (10) and/or the softness of the sealing edge (7) is such that the rolled-in sealing lip (10) and/or the sealing edge (7) folds over in the proximal direction at an expiratory pressure ≥ 30 mbar and remains **in that** position at such a pressure.

11. A tracheal cannula (1) according to one of claims 1 to 10 **characterised in that** on its outside the tube (2) has a flushing and/or suction opening (12) in a region between the proximal end (6b) of the ring cuff (6) and the distal end (6a) of the ring cuff (6), wherein the flushing and/or suction opening (12) can be connected to a flushing and/or suction device.

12. A tracheal cannula (1) according to one of claims 1 to 11 **characterised in that** on its outside the tube (2) has a flushing and/or suction opening (12) in a region between the distal end (6b) of the ring cuff (6) and the rolled-in sealing lip, wherein the flushing and/or suction opening (12) can be connected to a flushing and/or suction device.

13. A tracheal cannula (1) according to one of claims 1 to 12 **characterised in that** the tracheal cannula (1) has an axially air-permeable joint which connects the proximal end (3) and the distal end (4) of the tube (2) together and permits at least a limited mobility and in particular pivotability of the axis of the distal end (4) with respect to the axis of the proximal end (3).

14. Apparatus (13) for supplying respiratory air comprising a first tracheal cannula (1) and a second tracheal cannula (14) having a tube (15), wherein the tube (15) has a proximal end (16) with a proximal opening (16a) and a distal end (17) with a distal opening (17a), **characterised in that** the first tracheal cannula (1) is designed according to one of claims 1 to 13, wherein the tracheal cannula (1) with the elastic ring cuff (6) is of such a size and can be so introduced into the second tracheal cannula (14) that the ring cuff (6) projects beyond the distal end (17) of the second tracheal cannula (14).

15. Apparatus (13) for supplying respiratory air comprising a first tracheal cannula (1) and a second tracheal cannula (14) having a tube (15), wherein the tube (15) has a proximal end (16) with a proximal opening (16a) and a distal end (17) with a distal opening (17a), **characterised in that** the first tracheal cannula (1) is designed according to one of claims 1 to 13, wherein the second tracheal cannula (14) is of such a size and can be so introduced into the first tracheal cannula (1) with the elastic ring cuff (6) that a cuff (18) arranged on the outside of the tube (15) of the second tracheal cannula (14) projects beyond the distal end (17) of the tube (2) of the first tracheal cannula (1) with the elastic ring cuff (6).

## Revendications

1. Canule trachéale (1), en particulier canule pour trachéotomie ou tube endotrachéal, comportant un tube (2), dans laquelle le tube (2) présente une extrémité proximale (3) munie d'une ouverture proximale (3a), une extrémité distale (4) munie d'une ouverture distale (4a) et un dispositif d'étanchéité (5) agencé au niveau de sa périphérie extérieure à proximité de l'extrémité distale (4), dans laquelle le dispositif d'étanchéité (5) est un manchon annulaire (6) à pourtour annulaire périphérique, courbé vers l'extérieur et vers l'extrémité proximale (3) selon une forme d'entonnoir, dont l'extrémité distale (6a) entoure de manière étanche le tube (2) et dont l'extrémité proximale (6b) libre est réalisée sous la forme d'un bord d'étanchéité (7) périphérique présentant une élasticité en flexion, **caractérisée en ce que** le manchon annulaire (6) présente, de manière distale au-dessous de son extrémité proximale, en plus du bord d'étanchéité (7) situé de manière proximale au-dessus, une lèvre d'étanchéité (10) périphérique enroulée en direction de l'extrémité distale du manchon annulaire (6).

2. Canule trachéale (1) selon la revendication 1, **caractérisée en ce que** le manchon annulaire (6) en forme d'entonnoir est déformable par élasticité, où le bord d'étanchéité (7) élastique est précontraint radialement vers l'extérieur et/ou dans le sens circonférentiel.

3. Canule trachéale (1) selon la revendication 1 ou 2, **caractérisée en ce que** le bord d'étanchéité (7) élastique présente au moins une section de renforcement (8), au moins une section de renforcement (8) s'étendant le long du bord radialement intérieur du bord d'étanchéité (7) et/ou une section de renforcement (8) s'étendant parallèlement et à distance du bord radialement extérieur du bord d'étanchéité (7).

4. Canule trachéale (1) selon l'une des revendications 1 à 3, **caractérisée en ce que** le bord d'étanchéité (7) élastique présente au moins une section de renforcement (8) sous la forme d'une pluralité de nervures de renforcement (9) s'étendant de manière radiale, et/ou le bord d'étanchéité (7) élastique a la forme d'une lèvre d'étanchéité ou est un bourrelet d'étanchéité, de manière préférée toroïdal, en un matériau élastique souple.

5. Canule trachéale (1) selon l'une des revendications 1 à 4, **caractérisée en ce que** le bord d'étanchéité (7) élastique présente une dureté Shore A comprise entre 3 à 60, de manière préférée comprise entre 10 et 40.

6. Canule trachéale (1) selon l'une des revendications 1 à 5, **caractérisée en ce que** la périphérie radialement extérieure du bord d'étanchéité (7) présente un contour circulaire, un contour ovale, ou un contour en forme de D avec des coins arrondis, en particulier un contour s'approchant d'une section transversale typique de trachée.

7. Canule trachéale (1) selon l'une des revendications 1 à 6, **caractérisée en ce que** l'extrémité distale (6a) du manchon annulaire (6) se fond directement et de manière étanche dans le matériau du tube (2) et/ou **en ce que** le manchon annulaire (6) présente entre son extrémité proximale (6b) et son extrémité distale (6a) une section de transition (6c) incurvée dans le sens longitudinal du manchon annulaire (6).

8. Canule trachéale (1) selon l'une des revendications 1 à 7, **caractérisée en ce que** l'épaisseur de matière de la lèvre d'étanchéité (10) est dimensionnée de telle manière que la section transversale de la lèvre d'étanchéité (10) s'évase de manière radiale vers l'extérieur sous l'effet d'un flux de fluide se déplaçant d'un point distal vers un point proximal avec une pression ≥ 3 mbar, de manière préférée avec une pression ≥ 15 mbar.

9. Canule trachéale (1) selon la revendication 7 ou 8, **caractérisée en ce que**, vue en coupe transversale, la lèvre d'étanchéité (10) enroulée s'étend dans son état enroulé approximativement selon un arc de cercle ou de spirale inférieur à 360°, de manière préférée inférieur à 300° ou inférieur à 270°, mais supérieur à 180°, de manière préférée supérieur à 200° et de manière particulièrement préférée supérieur à 225° et/ou **en ce que** le manchon annulaire (6) présente, de manière proximale par rapport à la lèvre d'étanchéité (10), une pluralité de jours (11) dans la paroi annulaire de manchon, les jours (11) présentant de manière préférée un diamètre dans une plage comprise entre 0,2 mm et 2 mm, de manière préférée dans une plage comprise entre 0,5 mm et 1,5 mm.

10. Canule trachéale (1) selon l'une des revendications précédentes, **caractérisée en ce que** la souplesse de la lèvre d'étanchéité (10) enroulée et/ou la souplesse du bord d'étanchéité (7) est/sont conçue(s) de telle manière que la lèvre d'étanchéité (10) enroulée et/ou le bord d'étanchéité (7) est/sont replié(e)(s) dans le sens proximal pour une pression expiratoire ≥ 30 mbar et reste(nt) dans cette position pour une telle pression.

11. Canule trachéale (1) selon l'une des revendications 1 à 10, **caractérisée en ce que** le tube (2) présente sur son extérieur une ouverture de rinçage et/ou d'aspiration (12) dans une région située entre l'extrémité proximale (6b) du manchon annulaire (6) et l'extrémité distale (6a) du manchon annulaire (6), l'ouverture de rinçage et/ou d'aspiration (12) pouvant être reliée à un dispositif de rinçage et/ou d'aspiration.

12. Canule trachéale (1) selon l'une des revendications 1 à 11, **caractérisée en ce que** le tube (2) présente sur son extérieur une ouverture de rinçage et/ou d'aspiration (12) dans une région située entre l'extrémité distale (6a) du manchon annulaire (6) et la lèvre d'étanchéité enroulée, l'ouverture de rinçage et/ou d'aspiration (12) pouvant être reliée à un dispositif de rinçage et/ou d'aspiration.

13. Canule trachéale (1) selon l'une des revendications 1 à 12, **caractérisée en ce que** la canule trachéale (1) présente une articulation axialement perméable à l'air qui relie l'une à l'autre l'extrémité proximale (3) et l'extrémité distale (4) du tube (2) et permet au moins une mobilité limitée, et en particulier une capacité de pivotement, de l'axe de l'extrémité distale (4) par rapport à l'axe de l'extrémité proximale (3).

14. Dispositif d'alimentation en air respirable (13) présentant une première canule trachéale (1) et une seconde canule trachéale (14) comportant un tube (15), dans lequel le tube (15) présente une extrémité proximale (16) munie d'une ouverture proximale (16a) et une extrémité distale (17) munie d'une ouverture distale (17a), **caractérisé en ce que** la première canule trachéale (1) est conçue selon l'une des revendications 1 à 13, la canule trachéale (1) munie du manchon annulaire (6) élastique étant dimensionnée et pouvant être introduite dans la seconde canule trachéale (14) de telle manière que le manchon annulaire (6) fait saillie au-delà de l'extrémité distale (17) de la seconde canule trachéale (14).

15. Dispositif d'alimentation en air respirable (13) présentant une première canule trachéale (1) et une seconde canule trachéale (14) comportant un tube (15), dans lequel le tube (15) présente une extrémité proximale (16) munie d'une ouverture proximale (16a) et une extrémité distale (17) munie d'une ouverture distale (17a), **caractérisé en ce que** la première canule trachéale (1) est conçue selon l'une des revendications 1 à 13, la seconde canule trachéale (14) étant dimensionnée et pouvant être introduite dans la première canule trachéale (1) munie du manchon annulaire (6) élastique de telle manière qu'une coiffe (18) agencée sur l'extérieur du tube (15) de la seconde canule trachéale (14) fait saillie au-delà de l'extrémité distale (17) du tube (2) de la première canule trachéale (1) munie du manchon annulaire (6) élastique.
